# EUROPEAN PATENT SPECIFICATION

(11) **EP 3 305 914 B1**
(45) Date of publication and mention of the grant of the patent: **13.10.2021**
(21) Application number: 16803768.7
(22) Date of filing: 03.06.2016
(51) Int. Cl.: C12Q 1/68, G01N 33/68, G01N 33/50, C12Q 1/6883

(54) **USE OF SH3YL1 AS MARKER FOR NEPHROPATHY DIAGNOSIS**
VERWENDUNG VON SH3YL1 ALS MARKER FÜR DIE DIAGNOSE VON NEPHROPATHIEN
UTILISATION DE SH3YL1 EN TANT QUE MARQUEUR POUR LE DIAGNOSTIC D'UNE NÉPHROPATHIE

(30) Priority: 05.06.2015 KR 20150079672
(43) Date of publication of application: 11.04.2018
(73) Proprietor: Aptabio Therapeutics Inc., Yongin-si, Gyeonggi-do 16954 (KR)
(72) Inventor: CHA, Dae Ryong, Seoul 06943 (KR); KANG, Young Sun, Seoul 04421 (KR); CHA, Jin Joo, Seoul 06649 (KR); BAE, Yun Soo, Goyang-si Gyeonggi-do 10402 (KR); YOO, Jung-Yeon, Seoul 07225 (KR); MOON, Sung Hwan, Suwon-si Gyeonggi-do 16222 (KR); LEE, Soo Jin, Suwon-si Gyeonggi-do 16709 (KR)
(74) Representative: dompatent von Kreisler Selting Werner - Partnerschaft von Patent- und Rechtsanwälten mbB
(86) International application number: PCT/KR2016/005906
(87) International publication number: WO 2016/195415

(56) References cited:
- WO-A1-2008/129265
- CN-B- 102 154 478
- KR-A- 20110 137 278
- KR-B1- 101 240 208
- US-A- 5 654 158
- US-A1- 2012 004 119
- US-A1- 2014 155 397
- SHARON N. COX ET AL: "Altered modulation of WNT-[beta]-catenin and PI3K/Akt pathways in IgA nephropathy", KIDNEY INTERNATIONAL, vol. 78, no. 4, 1 August 2010 (2010-08-01) , pages 396-407, XP055525719, LONDON, GB ISSN: 0085-2538, DOI: 10.1038/ki.2010.138
- SHINYA NAKATANI ET AL: "Targeted Proteomics of Isolated Glomeruli from the Kidneys of Diabetic Rats: Sorbin and SH3 Domain Containing 2 Is a Novel Protein Associated with Diabetic Nephropathy", EXPERIMENTAL DIABETES RESEARCH, vol. 2011, 1 January 2011 (2011-01-01), pages 1-11, XP055525810, US ISSN: 1687-5214, DOI: 10.1155/2011/979354
- KOBAYASHI ET AL.: 'Dock4 forms a Complex with SH3YL1 and regulates Cancer Cell Migration' CELLULAR SIGNALLING vol. 26, no. 5, 01 May 2014, pages 1082 - 1088, XP055502598 DOI: 10.1016/J.CELLSIG.2014.01.027

## Description

### TECHNICAL FIELD

The present invention relates to the use of SH3YL1 as a marker for diabetic nephropathy diagnosis. Also described is a composition for nephropathy diagnosis, a microarray for nephropathy diagnosis, and a kit for nephropathy diagnosis, comprises a substance making it possible to measure the expression level of SH3YL1 gene or the level of a protein that is encoded by SH3YL1 gene.

### BACKGROUND ART

Among renal diseases, diabetic nephropathy that is one of diabetic vascular complications is the first leading cause of end stage renal disease (ESRD). In Korea, patients with ESRD caused by diabetes have steadily increased from 30.8% in 1996 to 42.3% in 2006 and 48.0% in 2013. This situation suggests that suggests that diet, exercise and drug therapies, which are common therapies known to date, are not appropriate and that various approaches to etiology, in addition to existing diabetes and diabetic nephropathy therapies are required.

Currently, renin-angiotensin system (RAS) inhibitors are mainly used to prevent nephropathy from being caused by diabetes. RAS inhibitors exhibits not only hemodynamic efficacy but also an excellent effect of protecting renal function by inhibiting the production of cytokine, chemokine, growth factor and the like which induce chronic inflammatory responses or fibrosis. However, the therapeutic effects of the RAS inhibitors are below expectations due to genetic propensities different between patients, side effects caused by long-term administration, and resistance to the therapeutic agents.

In addition, although extensive studies on the identification of the causes of nephropathy and diagnostic methods and therapeutic methods against nephropathy have been conducted over the past years, a definitive therapeutic agent capable of effectively treating nephropathy in early stages has not yet been developed. Thus, treating diabetic nephropathy by identifying at the early stage of diabetic nephropathy can be the most effective method.

Microalbuminuria (24-hr urine albumin of 30-300 mg/d or albumin excretion rate of 20-200 µg/min) is most frequently used as a biomarker that predicts the progression of diabetic nephropathy at the early stage and that predicts vascular complications other than nephropathy. According to existing monitoring studies, microalbuminuria is not only a risk factor that predicts reduced glomerular filtration rate, but also an independent risk factor that progresses to chronic renal failure. For such reasons, current therapeutic and diagnostic guidelines for diabetes recommend the measurement of microalbuminuria.

However, recent studies have reported that there is a limitation in using microalbuminuria alone as a marker for early diagnosing nephropathy and determining the progression of nephropathy.

The first reason is that frequency in natural disappearance of microalbuminuria in diabetic patients is high. Natural disappearance of microalbuminuria was shown at a frequency of about 21-64% in type 1 or type 2 diabetic patients with microalbuminuria. Furthermore, the results of large-scale 10-year follow-up studies on type 1 diabetic patients indicated that the frequency of patients who progressed to prominent proteinuria was 28% and the frequency of patients with the natural disappearance of microalbuminuria was as high as 40%. In addition, 5-year follow-up studies on type-1 diabetic patients who showed continued microalbuminuria in 2-3 measurements for 1 year have reported disappearance of microalbuminuria at a frequency of 64% (Tabaei BP et al., Diabetes Care, 24;1560-1566, 2001; Perkins BA et al., N Eng J Med, 348:2285-2293, 2003, Hovind P et al. BMJ, 328:1105, 2004; Steinke JM et al., Diabetes, 54:2164-2171,2005; De Boer IH et al., Arch Intern Med, 171:412-420, 2011; Gaede P et al., Nephrol Dial Transplant, 19:1784-2788, 2004; Araki S et al., Diabetes, 54:2983-2987, 2005; Yamada T et al., Diabetes Care, 28:2733-2738, 2005; RJ Maclsaac et al, Kidney International, 86:50-57, 2014).

The second reason is that the frequency of diabetic patients with reduced renal function (glomerular filtration rate <60ml/min/1.73m²) without microalbuminuria is high. According to studies conducted in early 1990s, the results of tissue examination on type 1 diabetic patients with reduced renal function without microalbuminuria indicated that a patient group who showed typical diabetic nephropathy was present and deterioration of renal function progressed in about 30% of type 1 and type 2 diabetic patients. In addition, follow-up studies on type 2 diabetic patients have indicated that deterioration of renal function preceded the occurrence of microalbuminuria (Tsalamandris C et al., Diabetes, 43:649-655, 1994; Yokoyama H et al., Nephrol Dial Transplant, 24:1212-1219, 2009; Molith ME et al., Diabetes Care, 33:1536-1543,2010; Kramer HJ et al., JAMA, 289:3273-3277, 2003).

Cox, S.N., Sallustio, F., Serino, G., Pontrelli, P., Verrienti, R., Pesce, F., Torres, D.D., Ancona, N., Stifanelli, P., Zaza, G. and Schena, F.P., 2010. Altered modulation of WNT-β-catenin and PI3K/Akt pathways in IgA nephropathy. Kidney international, 78(4), pp.396-407, discloses the expression level of SH3YL1 gene is lower in IgA nephropathy.

WO 2008/129265 A1 discloses diagnostic methods for diabetic nephropathy by using CCL18 gene/protein as the biomarker.

Accordingly, the present inventors have made extensive efforts to develop a novel method capable of early diagnosing nephropathy, and as a result, have found that the expression level of SH3YL1 gene in nephropathy patient's blood or the level of SH3YL1 protein in the blood significantly increases compared to that in non-nephropathy patient's blood, thereby completing the present invention.

### DISCLOSURE OF INVENTION

### TECHNICAL PROBLEM

It is an object of the present invention to provide a method for diagnosing diabetic nephropathy according to the expression level of SH3YL1 gene or the level of SH3YL1 protein.

Another object of the present invention is to provide a method for providing information required for nephropathy diagnosis comprising: measuring and detecting the expression level of SH3YL1 gene and/or the level of SH3YL1 protein.

Still another object of the present invention is the use of a microarray for diagnosing diabetic nephropathy by detecting the expression level of SH3YL1 gene and/or the level of SH3YL1 protein.

Yet another object of the present invention is to use a kit to measure the expression level of SH3YL1 gene and/or the level of SH3YL1 protein to early diagnose diabetic nephropathy.

### TECHNICAL SOLUTION

To achieve the above object, the present invention provides a method for diagnosing diabetic nephropathy comprising measuring the expression level of SH3YL1 gene or the level of SH3YL1 protein in a biological sample, characterized in that diabetic nephropathy is identified by an increased expression level of SH3YL1 gene or SH3YL1 protein in the sample compared to a normal control sample.

The present invention also provides a method for providing information required for nephropathy diagnosis comprising: treating a biological sample, extracted from a patient, with the above-described composition for nephropathy diagnosis to detect the expression level of SH3YL1 gene or the level of SH3YL1 protein in the sample.

The present invention also provides use of a microarray for nephropathy diagnosis, which comprises immobilized thereon a probe capable of hybridizing to SH3YL1 gene or a fragment thereof.

The present invention also provides use of a kit for nephropathy diagnosis, which comprises a primer pair capable of amplifying SH3YL1 gene or a fragment thereof, a probe capable of hybridizing to the SH3YL1 gene, or an antibody or aptamer that binds specifically to a protein encoded by the SH3YL1 gene.

The present invention also provides a method for diagnosis of nephropathy, comprising measuring the expression level of SH3YL1 gene or the level of SH3YL1 protein in a biological sample.

The embodiments of the present invention are reflected in independent claims 1, 4, 6 and 7. The preferred embodiments of the present invention are reflected in dependent claims 2, 3, and 5.

### BRIEF DESCRIPTION OF THE DRAWINGS

FIG. 1 shows the linear structure of SH3YL1.
FIG. 2 shows analysis results indicating that expression of SH3YL1 in cells is increased by LPS.
FIG. 3 shows the results of confirming the extracellular secretion of SH3YL1 caused by LPS stimulation in HPMEC cells.
FIG. 4 shows changes in SH3YL1 levels between patient groups.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods, which will be described below, are those well known and commonly employed in the art.

Conventionally, albuminurea, eGFR, UACR, blood creatine and the like have been used as markers for nephropathy diagnosis. However, diagnosis by these markers had a problem in that it is possible to diagnose nephropathy, only after 70% or more of the kidney is broken.

In the present invention, in order to identify either the expression levels of genes or the levels of proteins that are encoded by the genes showing a difference in expression between renal tissue and non-renal tissue were examined. As a result, it was found that SH3YL1 gene and/or SH3YL1 protein was overexpressed in renal tissue compared to in non-renal tissue.

Particularly, based on the finding that SH3YL1 gene and/or SH3YL1 protein is overexpressed in renal tissue, it was found that the SH3YL1 gene and/or the SH3YL1 protein can be used as a biomarker capable of diagnosing and predicting the onset of nephropathy, and that a substance capable of inhibiting the expression of SH3YL1 gene and/or the level of SH3YL1 protein can be used for prevention and treatment of nephropathy.

As used herein, the term "SH3YL1 protein" refers to a protein that comprises an amino acid sequence represented by SEQ ID NO: 1, or a protein that comprises an amino acid sequence having a sequence homology of at least 90%, preferably at least 95%, more preferably at least 99% to the amino acid sequence.

As used herein, the term "SH3YL1 gene" or "sh3yl1", which is a gene that encodes the amino acid sequence of "SH3YL1 protein", refers to a gene that comprises a nucleic acid sequence represented by SEQ ID NO: 2, or a gene that comprises a nucleic acid sequence having a sequence homology of at least 90%, preferably at least 95%, more preferably at least 99% to the amino acid sequence.

As used herein, the term "level of SH3YL1 protein" refers to the *in vivo* concentration or activity of SH3YL1 protein, and the term "expression level of SH3YL1 gene" refers to the *in vivo* expression level of "SH3YL1 gene".

As used herein, the term "diagnosis" refers identifying pathological conditions. For the purpose of the present invention, the term "diagnosis" refers detecting the progression of nephropathy by determining whether the nephropathy diagnostic marker was expressed. In addition, the term "diagnosis", as used herein, includes determining whether nephropathy developed and alleviation of nephropathy by determining whether the nephropathy diagnostic marker was expressed and the expression level of the nephropathy diagnostic marker.

Therefore, in one aspect, the present invention is directed to a composition for nephropathy diagnosis, comprising a substance making it possible to measure the expression level of SH3YL1 gene or the level of a protein that is encoded by the SH3YL1 gene.

In one example of the present invention, it was shown that, in the HAECs (human aortic endothelial cells) and HPMEC (human pulmonary microvascular endothelial cell) treated with the inflammation inducer LPS, expression of SH3YL1 gene increased and extracellular secretion of SH3YL1 protein increased.

In another example of the present invention, the expression level of SH3YL1 gene in the blood of nephropathy patient groups with different albuminuria values and the level of SH3YL1 protein in the blood were compared with those in a normal group. As a result, it could be seen that the expression level of SH3YL1 gene in the nephropathy patients significantly increased compared to that in the normal group.

In the present invention, the expression level of the SH3YL1 gene may be selected from the group consisting of the presence or absence of expression, the expression amount, and the expression pattern, and the level of the protein that is encoded by the SH3YL1 gene may be selected from the group consisting of the presence or absence, the expression amount, and the expression pattern of the protein.

In the present invention, the substance making it possible to measure the expression level of the SH3YL1 gene may be either a primer pair capable of amplifying the SH3YL1 gene or a probe capable of hybridizing to the SH3YL1 gene.

In the present invention, a method of measuring the expression amount of the SH3YL1 gene or the amount of the SH3YL1 protein may be selected from the group consisting of reverse transcriptase-polymerase chain reaction (RT-PCR), real time-polymerase chain reaction, Western blotting, Northern blotting, ELISA (enzyme linked immunosorbent assay), radioimmunoassay (RIA), radioimmunodiffusion, and immunoprecipitation assay.

In the present invention, "the level of SH3YL1 gene" preferably refers the mRNA expression level of the SH3YL1 gene, that is, the amount of mRNA, and substances capable of measuring the level comprise a primer or probe specific for the SH3YL1 gene. In the present invention, the primer or probe specific for the SH3YL1 gene may be a primer or probe capable of specifically amplifying the full length of the SH3YL1 gene represented by SEQ ID NO: 2 or a specific region of the gene. The primer or probe can be designed by a method that is well-known in the art.

As used herein, the term "primer" refers to a single-stranded oligonucleotide capable of acting as a point of initiation of template-directed DNA synthesis under appropriate conditions (i.e., in the presence of four different nucleoside triphosphates and an agent for polymerization) in an appropriate buffer and at a suitable temperature. The appropriate length of the primer may vary according to various factors, for example, temperatures and the intended use of the primer. Further, the primer need not be perfectly complementary to the exact sequence of a template, but should be sufficiently complementary to hybridize with the template. Thus, the primer in the present invention need not be perfectly complementary to the nucleotide sequence of the SH3YL1 gene (template) and should be sufficiently complementary to hybridize with the sequence of the gene. In addition, the primer according to the present invention can be used in a gene amplification reaction.

As used herein, the term "amplification reaction" refers to a reaction that amplifies nucleic acid molecules. Such amplification reactions of genes are well known in the art and include, for example, polymerase chain reaction (PCR), reverse-transcription polymerase chain reaction (RT-PCR), ligase chain reaction (LCR), transcription-mediated amplification (TMA), nucleic acid sequence-based amplification (NASBA), etc.

As used herein, the term "probe" refers to a linear oligomer of natural or modified monomers or linkages, including deoxyribonucleotides, ribonucleotides and the like, capable of specifically hybridizing with a target nucleotide sequence, whether occurring naturally or produced synthetically. The probe in the present invention may be single stranded. Preferably, it may be an oligodeoxyribonucleotide. The probe of the present invention may include naturally occurring dNMP (i.e., dAMP, dGMP, dCMP and dTMP), nucleotide analogs, or nucleotide derivatives. The probe in the present invention may also include ribonucleotides. For example, the probe of the present invention may include nucleotides with backbone modifications such as peptide (nucleic acid (PNA) (M. Egholm et al., Nature, 365:566-568 (1993)), phosphorothioate DNA, phosphorodithioate DNA, phosphoramidate DNA, amide-linked DNA, MMI-linked DNA, 2'-O-methyl RNA, alpha-DNA and methylphosphonate DNA, nucleotides with sugar modifications such as 2'-O-methyl RNA, 2'-fluoro RNA, 2'-amino RNA, 2'-O-alkyl DNA, 2'-O-allyl DNA, 2'-O-alkynyl DNA, hexose DNA, pyranosyl RNA, and anhydrohexitol DNA, and nucleotides having base modifications such as C-5 substituted pyrimidines (substituents including fluoro-, bromo-, chloro-, iodo-, methyl-, ethyl-, vinyl-, formyl-, ethynyl-, propynyl-, alkynyl-, thiazolyl-, imidazolyl-, pyridyl-), 7-deazapurines with C-7 substituents (substituents including fluoro-, bromo-, chloro-, iodo-, methyl-, ethyl-, vinyl-, formyl-, alkynyl-, alkenyl-, thiazolyl-, imidazolyl-, pyridyl-), inosine, and diaminopurine.

In the present invention, the substance making it possible to measure the level of the SH3YL1 protein may be a substance specific for the SH3YL1 protein, preferably an antibody or aptamer, but is not limited thereto. The substance can be used without any limitation as long as it is a substance that can bind specifically to the SH3YL1 protein.

In the present invention, the antibody is intended to include polyclonal, monoclonal and recombinant antibodies. The antibody also includes whole antibody molecules as well as antibody fragments. In the present invention, the antibody may be all types of immunoglobulin molecules (e.g., IgG, IgE, IgM, IgD, and IgA), and sub-types thereof (e.g., IgG1, IgG2, IgG3, IgG4, IgAl, and IgA2), but is not limited thereto. "Fragments of the antibody" according to the present invention means fragments at least having the ability to bind to an antigen. Fragments of the antibody include single-chain antibodies, diabodies, triabodies, tetrabodies, Fab fragments, F(ab')₂ fragments, Fd, scFv, domain antibodies, minibodies, scap (single chain antibody, scAb), derivatives in antibody constant regions, and synthetic antibodies based on protein scaffolds.

In the present invention, as described above, the SH3YL1 protein was identified as a marker protein capable of diagnosing nephropathy. Thus, an antibody against the protein can be easily produced using conventional techniques known to those skilled in the art. For example, a polyclonal antibody against the protein can be produced using a method widely known in the art by injecting SH3YL1 antigen into an animal, extracting blood from the animal, and separating serum containing the antibody from the blood. This polyclonal antibody can be produced from any animal species hosts, including goats, rabbits, sheep, monkeys, horses, pigs, cattle, dogs, and the like. A monoclonal antibody against the protein can be produced either using a hybridoma method (Kohler et al., European Jounral of Immunology, 6, 511-519, 1976) widely known in the art or using phage antibody library (Clacksonet al, Nature, 352, 624-628, 1991, Marks et al, J. Mol. Biol., 222:58, 1-597, 1991) technology. Furthermore, the antibody according to the present invention may include an intact antibody comprising two full-length light chains and two full-length heavy chains, as well as functional fragments of the antibody molecule. "Functional fragments of the antibody molecule" fragments having at least an antigen-binding function, and include Fab, F(ab'), F(ab')2 and Fv.

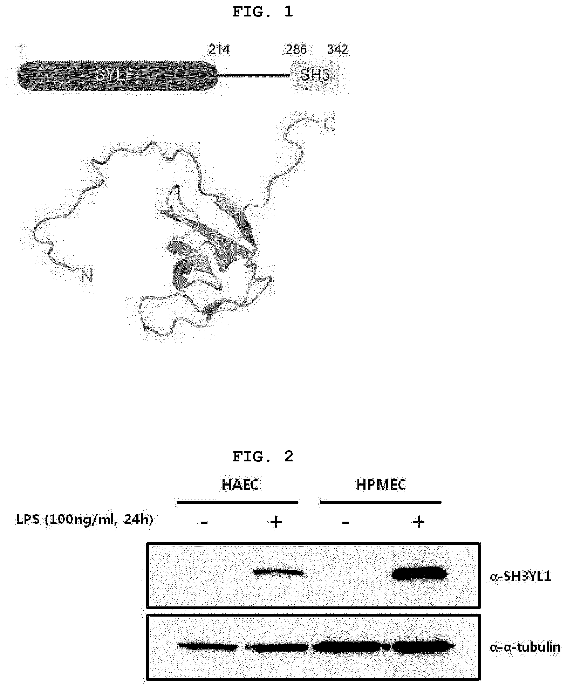

SH3YL1 (SH3 domain containing Ysc84-like1) is a protein having Src homologydomain3 (SH3) in the carboxyl terminal region and SYLF (SH3YL1, Ysc84p/Lsb4p, Lsb3p, and plant FYVE proteins that contain it) in the amino terminal region (FIG. 1). The nucleotide sequence of the SH3YL1 gene is represented by SEQ ID NO: 2, and the amino acid sequence of the SH3YL1 protein is represented by SEQ ID NO: 1.

The SH3 region of SH3YL1 recognizes a proline-rich region, and the SYLF region recognizes phosphatidylinositol 3,4,5-triphosphate (PI(3,4,5)P3) in the membrane. Due to this function, the SYLF region of SH3YL1 is known to recognize PI(3,4,5)P3 by stimulation of the growth factor PDGF to thereby regulate cellular migration (Hasegawa et al, JCB 193;901-916, 2011).

In another aspect, the present invention is directed to a method for providing information required for nephropathy diagnosis or prediction, the method comprising: treating a biological sample, extracted from a patient, with the above-described composition for nephropathy diagnosis to detect the expression level of SH3YL1 gene in the sample or the level of SH3YL1 protein in the sample.

In still another aspect, the present invention is directed to the use of SH3YL1 gene or SH3YL1 protein as a marker for nephropathy diagnosis.

In yet another aspect, the present invention is directed to a method for diagnosis of nephropathy, comprising measuring the expression level of SH3YL1 gene or the level of SH3YL1 protein in a biological sample.

As used herein, the term "biological sample" refers to a sample derived from a living organism, in which the expression level of SH3YL1 gene or the level of the protein according to the development or progression of nephropathy differs from that in a normal control group. Examples of the sample include, but are not limited to, tissues, cells, (whole) blood, serum, plasma, saliva and urine.

The measurement of the expression level of the SH3YL1 gene preferably include measuring the level of mRNA, and a method of measuring the level of mRNA includes reverse transcriptase-polymerase chain reaction (RT-PCR), real time-polymerase chain reaction, RNase protection assay, Northern blot analysis, and DNA chip, but is not limited thereto.

The measurement of the level of the SH3YL1 protein may be performed using an antibody that binds specifically to the SH3YL1 protein, preferably an antibody or aptamer specific for the SH3YL1 protein. In this case, the SH3YL1 marker protein in the biological sample and an antibody specific for the marker protein form a complex, that is, an antigen-antibody complex, and the amount of antigen-antibody complex formed may be quantitatively measured based on the magnitude of the signal of a detection label. This detection label may be selected from the group consisting of enzymes, fluorophores, ligands, luminophores, microparticles, redox molecules, and radioisotopes, but is not limited thereto. Analysis methods for measuring the protein levels include, but not limited to, Western blotting, ELISA, radioimmunoassay, radioimmunodiffusion, ouchterlony immunodiffusion, rocket immunoelectrophoresis, immunohistochemistry, immunoprecipitation assay, complement fixation assay, FACS, and protein chip assay.

Thus, the above-described detection methods according to the present invention makes it possible to determine the mRNA expression level of SH3YL1 gene or the level of SH3YL1 protein in a control group, and the mRNA expression level of SH3YL1 gene or the level of SH3YL1 protein in a patient suspected of having nephropathy, and can predict and diagnose the onset, progression stage or prognosis of nephropathy by comparing the expression level with that in the control group.

In an example of the present invention, blood was extracted from renal disease patients, including nephropathy patients, and serum was separated from the blood. The serum was subjected to ELISA assay using a SH3YL1-specific antibody to thereby measure the level of SH3YL1 protein in each patient's sample, and the measured values were compared with those in a control group.

In another aspect, the present invention is directed to a microarray for nephropathy diagnosis, which has immobilized thereon a probe capable of hybridizing to SH3YL1 gene or a fragment thereof.

In the microarray of the present invention, a primer, a probe or an antibody capable of measuring the expression level of SH3YL1 protein or a gene encoding the same is used as a hybridizable array element and is immobilized on a substrate. Preferred substrates may be any suitable rigid or semi-rigid supports, including, for example, membranes, filters, chips, slides, wafers, fibers, magnetic or nonmagnetic beads, gels, tubing, plates, polymers, microparticles and capillaries. The hybridizable array element is arranged and immobilized on the substrate, and this immobilization may be performed by a chemical binding method or a covalent binding method such as UV. For example, the hybridizable array element may be bound to a glass surface modified to contain an epoxy compound or an aldehyde group, and may also be bound to a polylysine-coated surface by UV irradiation. Furthermore, the hybridizable array elements may also be bound to the substrate through linkers (e.g. ethylene glycol oligomer and diamine).

Meanwhile, if a sample to be applied to the microarray of the present invention is nucleic acid, it may be labeled, and hybridized with the array element on the microarray. Various hybridization conditions are applicable, and detection and analysis of the extent of hybridization may be performed using various methods depending on the labels used.

In a still further aspect, the present invention provides a kit for nephropathy diagnosis, which contains a primer pair capable of amplifying SH3YL1 gene or a fragment thereof, a probe capable of hybridizing to the SH3YL1 gene, or an antibody or aptamer that binds specifically to a protein encoded by the SH3YL1 gene.

A composition for nephropathy diagnosis, which is included in the kit for nephropathy diagnosis according to the present invention, may comprise a primer, probe or antibody capable of measuring the expression level of SH3YL1 protein or a gene encoding the same, and the definition of the primer, probe and antibody is as described above.

If the kit for nephropathy diagnosis according to the present invention is to be applied to a PCR amplification process, the kit of the present invention may contain reagents required for PCR amplification, for example, buffer, DNA polymerase (e.g., thermally stable DNA polymerase obtained from Thermus aquaticus (Taq), Thermus thermophilus (Tth), Thermus filiformis, Thermis flavus, Thermococcus literalis or Pyrococcus furiosus(Pfu)), DNA polymerase cofactor and dNTPs. If the kit for nephropathy diagnosis according to the present invention is to be applied for immunoassay, the kit of the present invention may optionally contain secondary antibody and a labeled substrate. Furthermore, the kit according to the invention can be manufactured as a plurality of separate packages or compartments comprising the above mentioned reagent ingredients.

In a yet further aspect, the present invention is directed to a method for screening a substance for preventing or treating nephropathy, the method comprising the steps of: (a) bringing a candidate substance into contact with a cell that expresses SH3YL1 gene; (b) measuring the expression level of the SH3YL1 gene and/or the level of SH3YL1 protein; and (c) selecting the candidate substance as the substance for preventing or treating nephropathy, when the expression level of the SH3YL1 gene and/or the level of the SH3YL1 protein is decreased.

According to the method of the present invention, a sample to be analyzed may be brought into contact with a cell containing SH3YL1 gene or SH3YL1 protein. As used herein, the term "sample" or "candidate substance" refers to an unknown substance that is used in screening to examine whether it affects the expression level of the SH3YL1 gene, the level of the SH3YL1 protein or the activity of the SH3YL1 protein. The term "sample" or "candidate substance" may include chemical substances, nucleotides, antisense-RNAs, siRNAs (small interference RNAs), and natural product extracts, but is not limited thereof. Then, the expression amount of SH3YL1 gene and the level of SH3YL1 protein can be measured in a cell treated with a sample. As a result of the measurement, when the expression amount of SH3YL1 gene and the level of SH3YL1 protein are decreased, the sample can be determined to be a substance capable of preventing or treating nephropathy.

A method of measuring the expression amount of SH3YL1 gene and the level of SH3YL1 protein can be performed through various techniques well-known in the art. For example, the method measuring the expression amount of SH3YL1 gene and the level of SH3YL1 protein can be performed by using reverse transcriptase-polymerase chain reaction (RT-PCR), real time-polymerase chain reaction, Western blotting, Northern blotting, ELISA (enzyme linked immunosorbent assay), radioimmunoassay (RIA), radioimmunodiffusion, and immunoprecipitation assay.

Also described is an agent for treating nephropathy, which is a substance capable of reducing the expression level of SH3YL1 gene or the level of SH3YL1 protein, and a composition for treating nephropathy, which contains the same. In the present disclosure, preferred examples of substance capable of reducing the expression level of SH3YL1 gene include chemical substances, nucleotides, siRNA specific for the SH3YL1 gene, antisense nucleotides specific for the SH3YL1 gene, miRNA, and the like.

In the present disclosure, examples of the substance capable of decreasing the level of SH3YL1 protein may include antibodies, aptamers or chemical substances (small molecules), which are specific for the SH3YL1 protein.

The pharmaceutical composition described herein may further comprise a pharmaceutically acceptable carrier. As used herein, the term "pharmaceutically acceptable" refers to a physiologically acceptable composition which, when administered to human, will generally not cause allergic reactions, such as gastrointestinal disturbance, dizziness, and similar reactions. Examples of the pharmaceutically acceptable carrier include carriers for oral administration, such as lactose, starch, cellulose derivatives, magnesium stearate, and stearic acid, and carriers for parenteral administration, such as water, suitable oil, saline solution, aqueous glucose, and glycol. The composition of the present invention may further comprise a stabilizer and a preservative. Suitable stabilizers include antioxidants, such as sodium bisulphite, sodium sulphite and ascorbic acid. Suitable preservatives include benzalkonium chloride, methyl- or propyl-paraben, and chlorobutanol. Other pharmaceutically acceptable carriers can be found in Remington's Pharmaceutical Sciences, 19th ed., Mack Publishing Company, Easton, Pa., 1995.

The pharmaceutical composition described herein can be formulated into a suitable form together with the above pharmaceutically acceptable carrier according to any method known in the art. Specifically, the pharmaceutical composition can be formulated into various parenteral or oral dosage forms according to a conventional method. The parenteral dosage formulations typically include an injectable formulation, preferably, an isotonic solution or a suspension. The injectable formulation may be prepared using a suitable dispersing agent, wetting agent or suspending agent according to any method known in the art. For example, the injectable formulation can be prepared by dissolving necessary components in saline or buffer. Also, the oral dosage formulations include, but are not limited to, powders, granules, tablets, pills and capsules. The pharmaceutical composition formulated as described above may be administered in an effective amount by various routes, including oral, transdermal, subcutaneous, intravenous and intramuscular routes. The term "administration", as used herein, refers to the introduction of a predetermined substance into a patient using any suitable method. The substance may be administered via any general route, as long as it can reach target tissue.

As used herein, the term "effective amount" refers to an amount sufficient to achieve prevention or treatment when administered to a patient. The dose of the pharmaceutical composition of the present invention may vary depending on various factors, such as disease type and severity, age, body weight, sensitivity to drugs, type of current therapy, mode of administration, target cell, etc., and may be easily determined by those of ordinary skill in the art. The pharmaceutical composition of the present invention may also be administered in combination with conventional pharmaceutical agents, sequentially or simultaneously with the conventional pharmaceutical agents, and in single dose or multiple doses. Preferably, with all of the factors taken into account, the minimum dose required to achieve the maximum effect without side effects can be administered. Preferably, the composition may be administered several times a day at a dose of 1-10000 µg/kg weight/day, and more preferably 10-1000 mg/kg weight/day.

### EXAMPLES

Hereinafter, the present invention will be described in further detail with reference to examples. It will be obvious to a person having ordinary skill in the art that these examples are for illustrative purposes only and are not to be construed to limit the scope of the present invention.

### Example 1: Analysis of Intracellular SH3YL1 Level after LPS Treatment

SH3YL1 is present as a cytoplasmic protein and is involved in cellular migration. HAECs (human aortic endothelial cells, Lonza, cc-2535) and HPMECs (human pulmonary microvascular endothelial cells, Promo Cell, c-12281) were treated with the inflammation inducer LPS (100 ng/ml), and then the expression level of SH3YL1 in the cells was analyzed.

Specifically, under the conditions of 37°C and 5% CO₂, HAECs were cultured using Endothelial Cell Growth Medium 2 kit (cc-6162; Lonza), and HPMECs were cultured using Endothelial Cell Growth Medium MV kit (c-22120; Promo Cell). The cells were treated with LPS (100 ng/ml) for 24 hours, and then the levels of SH3YL1 in the cell lysates were analyzed.

As a result, as shown in FIG. 2, it could be seen that the expression of SH3YL1 in the cells was increased by LPS-induced inflammatory stimulation.

### Example 2: Examination of Whether or Not SH3YL1 Whose Expression was Increased Would Be Secreted Extracellularly

Whether or not SH3YL1 whose expression was increased by the inflammation inducer LPS would be secreted extracellularly was examined.

HPMEC cells were treated with LPS (100 ng/mL) at varying time points and cultured. The resulting culture was centrifuged, and the levels of SH3YL1 protein in the culture supernatant and the cell fraction were measured. Laemmli's SDS-PAGE sample buffer was added to the same amount of a sample whose concentration was measured by BCA protein assay (Pierce), and then the sample was boiled at 95°C for 10 minutes to prepare a loading sample. The sample was subjected to SDS-PAGE, and then electrotransferred to a nitrocellulose membrane and incubated in 5% skim milk for 30 minutes to remove non-specific bands. Next, the membrane was incubated overnight with primary antibody (diluted in 5% skim milk) at 4°C. Then, the membrane was washed three times with TBS-T buffer for 10 minutes each time, incubated with horseradish peroxidase-conjugated goat anti-mouse or rabbit IgG (santacruz biotechnology) at room temperature for 1 hours, and washed three times with TBS-T buffer for 10 minutes each time, and then a chemiluminescent image thereof was acquired using an imaging analyzer (LAS-3000, Fujifilm).

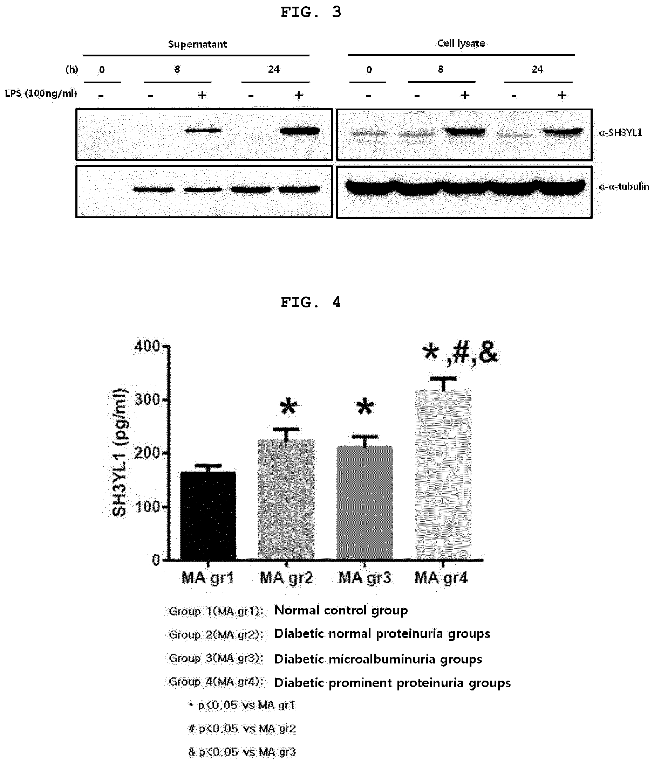

As a result, as shown in FIG. 3, it was shown that SH3YL1 protein was detected in the supernatant, indicating that the SH3YL1 protein was secreted extracellularly by LPS stimulation.

### Example 3: Examination of Increased Plasma SH3YL1 Protein Level in Diabetic Nephropathy Patients

Whether or not the level of SH3YL1 protein in the plasma of diabetic nephropathy patients would substantially increase was examined.

Cross-sectional studies on a total of 236 patients (65 normal persons and 171 type 2 diabetic patients) were performed by a single research institute.

The levels of plasma SH3YL1 protein in a total of 223 patients were quantitatively measured using an enzyme-linked immunosorbent assay (ELISA) kit (MBS936559; Myobiosource). This kit measures serum or plasma SH3YL1 levels based on the principle of sandwich ELISA.

To 96-well microplates to which a SH3YL1-specific antibody, five different concentrations of each of a standard substance, a blank substance, positive and negative control substances and a serum sample were added, allowed to react for 2 hours, and washed. Then, biotin-conjugated secondary antibody was added to the plate and allowed to react for 1 hour. After washing, horseradish peroxidase-conjugated solution together with streptavidin was added to the plate and allowed to react for 1 hours. After washing, a development reagent was added to the plate in a dark, and after 30 minutes, the absorbance at 450 nm was measured.

Using the absorbance values measured for five concentrations of the standard substance and the blank substance, a standard curve was obtained. Then, the absorbance value measured for each sample was converted into the concentration of SH3YL1 (pg/ml). Measurable concentration was 31.25 to 2000 pg/ml; intra-assay precision coefficient of variation (CV%) was <8%; and inter-assay precision (CV%) was <10%.

Diabetic patients were divided according to the stage of diabetic nephropathy into a normal proteinuria group (albumin-creatinine ratio: less than 30 µg/mg Cr), a microalbuminuria group (albumin-creatinine ratio: 30-300 µg/mg Cr), and a prominent proteinuria (more than 300 mg/mg Cr or 300 mg/day). Through Patient's medical records and consultation, data were checked, including underlying disease, sex, age, height, bodyweight, body mass index, and blood pressure.

Patient's blood samples were collected, and hemoglobin, leukocyte, platelet, C-reactive protein, blood urea (bun), creatinine and lipid levels in the samples were measured. The concentration of retinol binding protein 4 (RBP4) regarded as another marker of diabetic nephropathy was also measured using a kit (Phoenix).

For measurement of proteinuria, urine samples were collected, and microalbumin-to-creatinine ratio and protein-to-creatinine ratio in the urine samples were calculated. For calculation of glomerular filtration rate, the following CKD-EPI equation was used: glomerular filtration rate =141 x min (Scr/κ,1)α x max (Scr/κ,1)-1.209 x 0.993 age x 1.018 (female) x 1.159 (black person). κ=0.7 (female), κ=0.9 (male). α==0.329 (female), α=-0.411 (male). Insulin resistance was calculated using the following equation: HOMA-IR (homeostatic model assessment of insulin resistance=(fasting blood insulin (mU/I) x fasting blood glucose (mmol/l))/22.5).

For data analysis, IBBM SPSS Statistic 20 program was used, and two-tail test was performed (significant when P is 0.05). In order to normalize variables showing a non-normal distribution, analysis was performed after conversion to log value. For continuous data, t-test or Wilcoxon rank sum test, ANOVA test with post hoc analysis was performed depending on whether or not normality assumption would be satisfied. For dichotomous data, Chi-square test or Fisher's exact test was performed depending on the distribution of expected frequency. To confirm correlation, Pearson's correlation coefficient, linear and multiple regression analysis was used.

As a result, although there was no difference in age and male-to-female ratio between the groups, the renal function of all the groups with diabetic nephropathy was significantly lower than that of the control group, and the systolic blood pressure and insulin resistance values of the diabetic nephropathy groups were significantly higher than those of the control group (Table 1). In addition, a difference in C-reactive protein and lipid levels between the groups was not observed.

The level of SH3YL1 was significantly higher in the diabetic patient group than in the normal control group, and was significantly high in all the normal proteinuria groups, the microalbuminuria group and the prominent proteinuria group. In addition, it was shown that the level of SH3YL1 significantly increased in the patients showing prominent proteinuria (FIG. 4).

SH3YL1 may be influenced by baseline renal function, and thus the independent relevance thereof was examined. The results of simple correlation analysis indicated that SH3YL1 showed a positive correlation with proteinuria, microalbuminuria, RBP4 concentration, body mass index, systolic blood pressure and insulin resistance, and sowed no correlation with glomerular filtration rate (Tables 2 and 3). Even after correlation of age, sex and glomerular filtration rate, SH3YL1 level and microalbuminuria showed an independent positive correlation, and also showed a positive correlation with RBP4 concentration and a negative correlation with bone mass index (Table 4).

Through the above-described results, it could be found that the correlation between microalbuminuria and diabetic nephropathy, which are diagnostic and therapeutic targets of progression of diabetic nephropathy, and that the SH3YL1 protein can be used as a marker for predicting the progression of diabetic nephropathy.

**Table 1**

| | Normal control group n=65 | Diabetic normal proteinuria group n=66 | Diabetic microalbuminuria group n=52 | Diabetic prominent proteinuria n=53 | P value ANOVA |
|---|---|---|---|---|---|
| Age (years) | 54.8 ±12.3 | 55.4±10.9 | 56.5±13.5 | 55.6±12.0 | 0.909 |
| sex: male (%) | 29(45) | 32(48) | 22 (42) | 25 (47) | |
| Glomerular filtration rate | 96.2±22.9^{A} | 88.8±28.1^{B} | 83.6±34.2^{B} | 82.3±28.6^{C} | 0.035 |
| Proteinuria excretion rate | 335.1±427.5^{A} | 337.1±340.4^{A} | 822.0±1584. 4^{A} | 5588874.9± 143787^{B} | <0.00 1 |
| Microalbuminuria excretion rate | 10.87±7.116 | 9.87±5.234 | 70.23±57.07 7 | 1893.05±17 29.600 | <0.00 1 |
| Body mass index | 23.9±3.67 | 23.9±3.00 | 24.6±2.94 | 22.9±4.57 | 0.125 |
| Systolic blood pressure | 116.6±14.03^{A} | 124.7±13.69^{B} | 133.5±18.88 ^{c} | 139.8±18.3 4^{C} | <0.00 1 |
| Diastolic blood pressure | 73.4±11.91 | 72.8±10.19 | 78.8±11.46 | 78.8±12.76 | 0.010 |
| Insulin resistance HOMA-IR | 1.62±1.218^{A} | 7.04±5.170^{B} | 5.12±3.778^{B} | 6.11±7.886 ^{B} | <0.00 1 |
| C-reactive protein | 0.26 ±0.339 | 0.37 ±0.321 | 0.58 ±1.175 | 0.46 ±0.981 | 0.214 |
| Fasting blood glucose | 98.5 ±11.13^{A} | 141.7 192.25^{B} | 122.1 145.01^{C} | 122.1 ±46.39^{C} | 0.001 |
| 2 hours postprandial blood glucose | 111.5 ±29.78^{A} | 178.2 ±104.62^{B} | 154.5 ±75.89^{B} | 168.1 ±105.28^{B} | <0.00 1 |
| Total cholesterol | 189.2±38.87 | 195.9 ±45.65 | 186.6 ±31.13 | 186.3 ±36.63 | 0.507 |
| LDL cholesterol | 116.7 ±38.46 | 117.6 ±36.79 | 113.5 ±32.08 | 111.5 ±37.9 | 0.812 |
| HDL cholesterol | 48.2 ±14.85 | 44.3 ±12.62 | 44.1 ±10.25 | 47.7 ±19.68 | 0.329 |
| Triglyceride | 127.2 ±78.23 | 152.5 ±106.93 | 147.6 ±99.21 | 153.4 ±123.34 | 0.460 |

| | | | | | |
|---|---|---|---|---|---|
| 1) There was no statistical difference between the same capital letters in post-analysis. | | | | | |

**Table 2**

| | Pearson correlation | Significance probability (two-sided) |
|---|---|---|
| Body mass index | -0.168 | 0.016 |
| 2 hours postprandial blood glucose | 0.151 | 0.034 |
| Systolic blood pressure | 0.214 | 0.003 |
| Insulin resistance | 0.259 | <0.001 |
| Microalbuminuria excretion rate (log) | 0.306 | <0.001 |
| Proteinuria excretion rate (log) | 0.334 | <0.001 |
| RBP4 | 0.358 | <0.001 |

| | | |
|---|---|---|
| 1) Dependent variable: SH3YL1 | | |

**Table 3**

| | Non-standardized coefficient (standard error) | Standardized coefficient | Significance probability |
|---|---|---|---|
| Body mass index | -0.018(0.007) | -0.168 | 0.017 |
| Systolic blood pressure | 0.003(0.002) | 0.159 | 0.030 |
| Insulin resistance | 0.011(0.005) | 0.164 | 0.020 |
| Microalbuminuria excretion rate (log) | 0.126(0.026) | 0.315 | <0.001 |
| Proteinuria excretion rate (log) | 0.088(0.022) | 0.263 | <0.001 |
| RBP4 | 9.724E^{- 0.5}(2.1596E^{-0.6}) | 0.292 | <0.001 |
| Glomerular filtration rate | -0.001(0.001) | -0.055 | 0.417 |

| | | | |
|---|---|---|---|
| 1) Dependent variable: SH3YL1 (log) | | | |

**Table 4**

| | Non-standardized coefficient (standard error) | Standardized coefficient | Significance probability |
|---|---|---|---|
| Microalbuminuria excretion rate (log) | 0.123(0.030) | 0.334 | <0.001 |
| RBP4 | 7.029E^{-0.5} (0.001) | 0.228 | 0.008 |
| Body mass index | -0.019(0.009) | -0.180 | 0.027 |

| | | | |
|---|---|---|---|
| 1) Dependent variable: SH3YL1 (log) 2) Correction factors: proteinuria excretion rate (log) (log), glomerular filtration rate, C-reactive protein, 2 hours postprandial blood glucose, systolic blood pressure, age, sex. | | | |

**Table 5**

| SH3YL1 | | |
|---|---|---|
| Amino acid sequence | | SEQ ID NO: 1 |
| | | |
| Nucleotide sequence | | SEQ ID NO: 2 |
| | | |

### INDUSTRIAL APPLICABILITY

According to the present invention, the effect of promptly and accurately diagnosing nephropathy in early stages is obtained, and accurate diagnosis can be achieved only using patient's blood, and furthermore, a target for the development of a nephropathy therapeutic agent can be provided.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims.
## Claims

1. Method for diagnosing diabetic nephropathy comprising measuring the expression level of SH3YL1 gene or the level of SH3YL1 protein in a biological sample, **characterized in that** diabetic nephropathy is identified by an increased expression level of SH3YL1 gene or SH3YL1 protein in the sample compared to a normal control sample.

2. The diagnostic method of claim 1, wherein the SH3YL1 gene expression level is measured by either a primer pair capable of amplifying the SH3YL1 gene or a probe capable of hybridizing to the SH3YL1 gene.

3. The diagnostic method of claim 1, wherein the SH3YL1 protein level is measured by an antibody or aptamer binding specifically to a protein that is encoded by the SH3YL1 gene.

4. A method for providing information required for diabetic nephropathy diagnosis or prediction comprising: treating a biological sample with a substance selected from the group consisting of a primer pair capable of amplifying SH3YL1 gene or a fragment thereof; a probe capable of hybridizing to the SH3YL1 gene; and an antibody or aptamer that binds specifically to a protein encoded by the SH3YL1 gene to detect the SH3YL1 gene expression level or the SH3YL1 protein level in the sample.

5. The method of claim 4, wherein the biological sample is selected from the group consisting of tissues, cells, blood, serum, plasma, saliva and urine.

6. Use of a microarray for diabetic nephropathy diagnosis in the method according to claim 1, wherein the microarray comprises immobilized thereon a probe capable of hybridizing to SH3YL1 gene or a fragment thereof.

7. Use of a kit for diabetic nephropathy diagnosis in the method according to claim 1, wherein the kit comprises a substance selected from the group consisting of:
a primer pair capable of amplifying SH3YL1 gene or a fragment thereof;
a probe capable of hybridizing to the SH3YL1 gene; and an antibody or aptamer that binds specifically to a protein encoded by the SH3YL1 gene.

## Patentansprüche

1. Verfahren zum Diagnostizieren einer diabetischen Nephropathie, umfassend das Messen des Expressionsniveaus des SH3YL1-Gens oder des Niveaus des SH3YL1-Proteins in einer biologischen Probe, **dadurch gekennzeichnet, dass** eine diabetische Nephropathie durch ein im Vergleich zu einer normalen Kontrollprobe erhöhtes Expressionsniveau des SH3YL1-Gens oder des SH3YL1-Proteins in der Probe identifiziert wird.

2. Diagnoseverfahren gemäß Anspruch 1, wobei das Expressionsniveau des SH3YL1-Gens entweder durch ein Primerpaar, das das SH3YL1-Gen amplifizieren kann, oder eine Sonde, die mit dem SH3YL1-Gen hybridisieren kann, gemessen wird.

3. Diagnoseverfahren gemäß Anspruch 1, wobei das Niveau des SH3YL1-Proteins durch einen Antikörper oder ein Aptamer, das spezifisch an ein Protein bindet, welches von dem SH3YL1-Gen codiert wird, gemessen wird.

4. Verfahren zum Bereitstellen von Informationen, die für die Diagnose oder Vorhersage einer diabetischen Nephropathie erforderlich sind, umfassend: Behandeln einer biologischen Probe mit einer Substanz, die aus der Gruppe ausgewählt ist, die aus einem Primerpaar, das das SH3YL1-Gen oder ein Fragment davon amplifizieren kann, einer Sonde, die mit dem SH3YL1-Gen hybridisieren kann, und einem Antikörper oder einem Aptamer, das spezifisch an ein Protein bindet, welches von dem SH3YL1-Gen codiert wird, besteht, zum Nachweisen des Expressionsniveau des SH3YL1-Gens oder des Niveaus des SH3YL1-Proteins in der Probe.

5. Verfahren gemäß Anspruch 4, wobei die biologische Probe aus der Gruppe ausgewählt ist, die aus Geweben, Zellen, Blut, Serum, Plasma, Speichel und Urin besteht.

6. Verwendung eines Mikroarrays für die Diagnose einer diabetischen Nephropathie in dem Verfahren gemäß Anspruch 1, wobei auf dem Mikroarray eine Sonde immobilisiert ist, die mit dem SH3YL1-Gen oder einem Fragment davon hybridisieren kann.

7. Verwendung eines Kits für die Diagnose einer diabetischen Nephropathie in dem Verfahren gemäß Anspruch 1, wobei der Kit eine Substanz umfasst, die aus der Gruppe ausgewählt ist, die aus
einem Primerpaar, das das SH3YL1-Gen oder ein Fragment davon amplifizieren kann;
einer Sonde, die mit dem SH3YL1-Gen hybridisieren kann; und
einem Antikörper oder Aptamer, das spezifisch an ein Protein bindet, welches von dem SH3YL1-Gen codiert wird,
besteht.

## Revendications

1. Procédé pour diagnostiquer une néphropathie diabétique, comprenant l'étape consistant à mesurer le niveau d'expression du gène SH3YL1 ou le niveau de la protéine SH3YL1 dans un échantillon biologique, **caractérisé en ce que** la néphropathie diabétique est identifiée par un niveau élevé de l'expression du gène SH3YL1 ou de la protéine SH3YL1 dans l'échantillon par rapport à un échantillon de contrôle normal.

2. Procédé diagnostique selon la revendication 1, dans lequel ledit niveau d'expression du gène SH3YL1 est mesuré par soit une paire d'amorces capable d'amplifier le gène SH3YL1, ou une sonde capable de s'hybrider avec le gène SH3YL1.

3. Procédé diagnostique selon la revendication 1, dans lequel ledit niveau de la protéine SH3YL1 est mesuré par un anticorps ou aptamère se liant spécifiquement à une protéine encodée par le gène SH3YL1.

4. Procédé pour fournir des informations nécessaires pour le diagnostic ou la prédiction d'une néphropathie diabétique, comprenant l'étape consistant à traiter un échantillon biologique avec une substance choisie dans le groupe consistant en une paire d'amorces capable d'amplifier le gène SH3YL1 ou un fragment de celui-ci, une sonde capable de s'hybrider avec le gène SH3YL1, et un anticorps ou aptamère se liant spécifiquement à une protéine encodée par le gène SH3YL1, pour détecter le niveau d'expression du gène SH3YL1 ou le niveau de la protéine SH3YL1 dans l'échantillon.

5. Procédé selon la revendication 4, dans lequel ledit échantillon biologique est choisi dans le groupe consistant en tissus, cellules, sang, sérum, plasma, salive, et urine.

6. Utilisation d'un microréseau pour le diagnostic d'une néphropathie diabétique dans le procédé selon la revendication 1, dans lequel ledit microréseau comprend une sonde capable de s'hybrider avec le gène SH3YL1 ou avec un de ses fragments, immobilisé dessus.

7. Utilisation d'une trousse pour le diagnostic d'une néphropathie diabétique dans le procédé selon la revendication 1, dans lequel ladite trousse comprend une substance choisie dans le groupe consistant en
une paire d'amorces capable d'amplifier le gène SH3YL1 ou un fragment de celui-ci,
une sonde capable de s'hybrider avec le gène SH3YL1, et
un anticorps ou aptamère se liant spécifiquement à une protéine encodée par le gène SH3YL1.


<110> APTABIO THERAPEUTICS INC.
<120> USE of SH3YL1 As Diagnostic Marker for Nephropathy
<130> PP-B1749
<150> KR 10-2015-0079672
   <151> 2015-06-05
<160> 2
<170> KopatentIn 2.0
<210> 1
   <211> 342
   <212> PRT
   <213> Homo sapience
<400> 1
   Met Asn Asn Pro Ile Pro Ser Asn Leu Lys Ser Glu Ala Lys Lys Ala
   1               5                   10                  15
   Ala Lys Ile Leu Arg Glu Phe Thr Glu Ile Thr Ser Arg Asn Gly Pro
               20                  25                  30
   Asp Lys Ile Ile Pro Ala His Val Ile Ala Lys Ala Lys Gly Leu Ala
           35                  40                  45
   Ile Leu Ser Val Ile Lys Ala Gly Phe Leu Val Thr Ala Arg Gly Gly
       50                  55                  60
   Ser Gly Ile Val Val Ala Arg Leu Pro Asp Gly Lys Trp Ser Ala Pro
   65                  70                  75                  80
   Ser Ala Ile Gly Ile Ala Gly Leu Gly Gly Gly Phe Glu Ile Gly Ile
                   85                  90                  95
   Glu Val Ser Asp Leu Val Ile Ile Leu Asn Tyr Asp Arg Ala Val Glu
               100                 105                 110
   Ala Phe Ala Lys Gly Gly Asn Leu Thr Leu Gly Gly Asn Leu Thr Val
           115                 120                 125
   Ala Val Gly Pro Leu Gly Arg Asn Leu Glu Gly Asn Val Ala Leu Arg
       130                 135                 140
   Ser Ser Ala Ala Val Phe Thr Tyr Cys Lys Ser Arg Gly Leu Phe Ala
   145                 150                 155                 160
   Gly Val Ser Leu Glu Gly Ser Cys Leu Ile Glu Arg Lys Glu Thr Asn
                   165                 170                 175
   Arg Lys Phe Tyr Cys Gln Asp Ile Arg Ala Tyr Asp Ile Leu Phe Gly
               180                 185                 190
   Asp Thr Pro Arg Pro Ala Gln Ala Glu Asp Leu Tyr Glu Ile Leu Asp
           195                 200                 205
   Ser Phe Thr Glu Lys Tyr Glu Asn Glu Gly Gln Arg Ile Asn Ala Arg
       210                 215                 220
   Lys Ala Ala Arg Glu Gln Arg Lys Ser Ser Ala Lys Glu Leu Pro Pro
   225                 230                 235                 240
   Lys Pro Leu Ser Arg Pro Gln Gln Ser Ser Ala Pro Val Gln Leu Asn
                   245                 250                 255
   Ser Gly Ser Gln Ser Asn Arg Asn Glu Tyr Lys Leu Tyr Pro Gly Leu
               260                 265                 270
   Ser Ser Tyr His Glu Arg Val Gly Asn Leu Asn Gln Pro Ile Glu Val
           275                 280                 285
   Thr Ala Leu Tyr Ser Phe Glu Gly Gln Gln Pro Gly Asp Leu Asn Phe
       290                 295                 300
   Gln Ala Gly Asp Arg Ile Thr Val Ile Ser Lys Thr Asp Ser His Phe
   305                 310                 315                 320
   Asp Trp Trp Glu Gly Lys Leu Arg Gly Gln Thr Gly Ile Phe Pro Ala
                   325                 330                 335
   Asn Tyr Val Thr Met Asn
               340
<210> 2
   <211> 1029
   <212> DNA
   <213> Homo sapience
<400> 2
   atgaataacc ctataccttc caatttgaaa tcagaagcaa aaaaggctgc caaaatatta        60
   agagaattca cagaaataac ttccagaaat ggacctgata agatcattcc tgctcacgta       120
   attgcgaagg ctaaaggcct tgcaattctg tctgtgatca aagccgggtt cctggtgact       180
   gccagaggag gcagcgggat tgtagtggcg cgccttccag atggaaaatg gtctgcaccc       240
   tcagccattg ggatagctgg ccttggtgga ggatttgaaa taggaattga ggtatcagac       300
   ttggtgataa ttctgaatta tgaccgtgct gtagaagctt ttgcaaaagg cggaaatctg       360
   accctcggag ggaacttgac tgtggcggtt gggcccttgg gaaggaactt ggaaggaaac       420
   gtggccctga gaagctccgc tgccgtcttc acgtactgca agtcaagggg actctttgca       480
   ggcgtgtctt tagaagggag ctgtttgatt gaaaggaaag aaactaatag aaaattttat       540
   tgtcaagata tccgagctta tgacatttta tttggagata caccgcggcc tgctcaagcc       600
   gaagatcttt atgaaattct tgattccttt actgaaaagt atgaaaatga aggacaacga       660
   atcaatgcaa gaaaagcagc aagggagcag aggaagtctt ctgctaaaga attacctcca       720
   aagccattgt caagaccaca gcagtcatct gcaccagtcc agctgaactc tggctctcaa       780
   agtaacagaa atgaatataa gctctatcct ggactttcca gctatcatga gagagttggc       840
   aatttgaatc aacccataga agtgacagcg ctgtattcat ttgaaggaca gcagcctggg       900
   gatttgaatt ttcaagctgg agacagaatc acagttatat caaaaacaga ttcacatttt       960
   gattggtggg aaggaaaact tcgaggtcaa actggcattt ttccagccaa ctacgtaacc      1020
   atgaattaa                                                              1029